Europäisches Patentamt

European Patent Office

Office européen des brevets

(19)

(11) Publication number: **0 299 715**
**A2**

## (12) EUROPEAN PATENT APPLICATION

(21) Application number: 88306349.7

(22) Date of filing: 12.07.88

(51) Int. Cl.4: **C12P 13/22 , C12N 9/88 ,**
**C12N 9/96**

(30) Priority: 13.07.87 JP 172863/87
27.08.87 JP 211355/87

(43) Date of publication of application:
18.01.89 Bulletin 89/03

(84) Designated Contracting States:
AT BE CH DE ES FR GB IT LI NL

(71) Applicant: MITSUI TOATSU CHEMICALS INC.
No. 2-5, Kasumigaseki 3-chome
Chiyoda-Ku Tokyo 100(JP)

(72) Inventor: Miyahara, Shoichiro
1413-1343, Shiomidai 1-4, Isogo-ku
Yokohama-shi Kanagawa-ken(JP)
Inventor: Yanaka, Makoto
521, Kamishirakawa-machi 2-308
Oomuta-shi Fukuoka-ken(JP)
Inventor: Matsumoto, Toshio
300-422, Hirabaru-machi
Oomuta-shi Fukuoka-ken(JP)
Inventor: Miyahara, Tooru
9-3, Torizuka-machi
Oomuta-shi Fukuoka-ken(JP)
Inventor: Sakaguchi, Akio
2-308, Kamishirakawa-machi
Oomuta-shi, Fukuoka-ken(JP)
Inventor: Nitta, Kazunari
1874-28, Oaza-Misaki
Oomuta-shi, Fukuoka-ken(JP)

(74) Representative: Hayward, Denis Edward Peter
et al
Lloyd Wise, Tregear & Co. Norman House
105-109 Strand
London WC2R 0AE(GB)

(54) **Process for preparing L-tryptophan and process for preparing a stable aqueous enzyme solution.**

(57) A method of preparing L-tryptophan by reacting indole and serine and using an aqueous solution of enzymes which are extracted from cells. After the reaction, the pH value of the reaction mass is adjusted to above 10 by ammonia. The L-tryptophan can be dissolved in the solution in a high concentration, so purification and separation are facilitated and the loss of L-tryptophan minimalized. The preparation of a stable aqueous solution of enzymes is also provided wherein an extracted enzyme solution is heated above 40°C.

EP 0 299 715 A2

# PROCESS FOR PREPARING L-TRYPTOPHAN AND PROCESS FOR PREPARING A STABLE AQUEOUS ENZYME SOLUTION

The present invention relates to a method of enzymatic preparation of L-tryptophan by reacting indole with serine and a method for preparing a stable aqueous enzyme solution.

L-tryptophan is one of the essential amino acids and is a useful compound in the field of pharmaceuticals, health foods and feed additives. Several processes of preparing L-tryptophan are known, such as the fermentation method using glucose as a starting material, the so-called semi-fermentation method which uses anthranilic acid as a starting material, the method utilizing the enzymatic condensation reaction between indole and serine and the method of utilizing enzymes with indole, pyruvic acid and ammonia. These enzymatic reactions are catalyzed by tryptophan synthase.

It is well known that enzymes such as tryptophan synthase used for the enzymatic reaction are relatively unstable when these enzymes are extracted from the strain in the liquid state. Consequently, in order to obtain stable enzymes, the purification of the enzymes is required. The operation has several steps and is complicated. The purification technique, while perhaps feasible on a laboratory scale, has significant disadvantages when practiced on a large scale. Therefore, the production of tryptophan is carried out by using whole cells of micro-organisms having high enzyme activity. (See U.S. Patent No. 4,335,209).

However, when the enzyme source is used in the form of cells, there is difficulty in the disposing treatment of the used cells which become waste after the reaction is ended. It is known to remove the waste cells from the reaction mass by adjusting the pH value to a level not more than 4, heating the mass using activated carbon as core material onto which the cells are aggregated and adsorbed. Thereafter, solid-liquid separation is undertaken. (See "Hakko to Kogyo", Vol. 40, No. 10, 906-916, GB No. 2,152,031).

It is necessary that during the separation of the waste cells, the operation is to be conducted under the condition that the amino acid is completely dissolved. However, since the solubility of L-tryptophan in water is low, a great amount of water at a pH of 4 to 5 is required to dissolve the L-tryptophan. Therefore, the volumetric efficiency is poor and during the separation steps, L-tryptophan is attached or adsorbed onto the activated carbon which is the core material of the aggregation. Thus, this known process results in a significant loss of L-tryptophan.

The aqueous enzyme solution can be prepared by fractionating the tryptophan synthase using ammonium sulfate in the presence of phenylurethane sulfonyl fluoride a protease inhibitor to separate other proteins. The fractionated enzymes are dissolved and dialyzed with an appropriate buffer solution to remove ammonium sulfate and phenyl-methane sulfonyl fluoride. Thus, this preparation of the enzyme solution is not sufficient for industrial levels of operation. (See J. of Biol.Chem. Vol. 249 [1974] No. 24,7756-7763). Thus, it is necessary to remove the protease inhibitor.

It is one object of the invention to provide an improved process for preparing L-tryptophan by reacting indole and L-serine using an aqueous enzyme solution. It is another object of the invention to provide a method of preparing a stable aqueous enzyme solution.

The inventors, based on the disadvantages of using enzymes in the form of cells in a process of preparing L-tryptophan by reacting indole and serine, have investigated intensively the use of an aqueous enzyme solution. As a result, they have found that when the reaction is conducted by using an aqueous enzyme solution extracted from cells, which is to be prepared according to the present invention or known processes, and after finishing the reaction, the pH value of the reaction mass is adjusted to above 10 by the addition of ammonia. The accumulated L-tryptophan in the reaction mass dissolves completely in the aqueous portion which becomes a homogeneous solution that can be easily separated from the organic solvent residue and subjected to further purification steps. One important advantage of this method is that flocculation of waste cells does not occur which would cause the formation of two separate phases.

After the purification treatment, L-tryptophan is recovered by heating the solution to remove ammonia and condensing the aqueous solution. Thus, the present invention is a method of enzymatically preparing L-tryptophan by reacting indole and serine, which comprises conducting the reaction in an aqueous enzyme solution extracted from cells, then after finishing the reaction, adjusting the pH value of the reaction mass above 10 by adding ammonia to dissolve the L-tryptophan so as to form a homogeneous solution. The solution is subjected to purification steps and, thereafter the solution is heated to release the ammonia and the L-tryptophan is recovered.

As for the stable enzyme solution, it has been found that after extracting enzymes from cells, and heating the extracted mass above 40°C, a purified and stable solution can be obtained. That is, another object of the present invention is a method of preparing an aqueous solution of tryptophan synthase or tryptophanase which comprises: (a) after culturing cells of microorganism strains capable of producing tryptophan synthase or tryptophanase, rupturing the cells to extract enzymes from the cells; (b) heating said extracted enzymes above 40°C and to a temperature where the activity of proteins other than the tryptophan synthase or tryptophanase are insolubilized or deactivated; and (c) removing said insoluble protein and the residue of the cells.

As the microorganisms capable of producing the enzymes, which are used for the reaction of indole and serine, the microorganisms producing tryptophan synthase can be used. For example, Escherichia coli MT10232 (Ferm BP-19), Escherichia coli MT10242 (Ferm BP-20) and Neurospora crassa ATCC 14692 can be used. It is preferable to use the aqueous solution of tryptophan synthase extracted from Escherichia coli. An aqueous solution of tryptophanase extracted from the strain such as Aerobacter aerogenes can be used which is described in U.S. Patent No. 4,335,209, the contents of which are incorporated by reference.

For culturing the tryptophanase and tryptophan synthase producing microorganism, any synthetic or natural culture media can be used so far as it contains a carbon source, a nitrogen source, an inorganic substance and, if necessary, a small amount of a nutrient. For example, for the carbon source, a sugar can be used such as sucrose, glucose and molasses. Multivalent alcohols, such as glycerol, and organic acids, such as citric acid, are also available as the carbon source.

For the nitrogen source, there can be used various materials, such as ammonia, ammonium chloride, ammonium sulfate and ammonium phosphate.

For the inorganic substance, potassium dihydrogen phosphate, dipotassium hydrogen phosphate, magnesium sulfate, iron and manganese can be used.

For the nutrient, amino acids, vitamins, nucleic acids, yeast extract, meat extract and polypepton can be used.

As to the preparation of the stable aqueous solution of enzymes, the following process can be used.

For the cell rupturing step, the cultivated media just after cultivation or diluted to buffer after collecting cells by a centrifuge method is preferably subjected to physical or mechanical pulverization, such as ultrasonic treatment, milling and pressure shearing. Alternatively, Dyno-Mill KDL (Willy A. Bachofen) for milling, French Pressure Cell press (SLM AMINCO) or High Pressure Homogenizer (Bran & Luebbe) for pressure shearing, can be used.

The rupturing conditions depend on the equipment used, but it is preferred that rupturing be conducted under the condition that the concentration of the cells is 5 to 100 g/l, more preferably 30 to 60 g/l. The pH can be 6 to 10, and the temperature is desired to be below 30°C to prevent the deactivation of the enzymes, more preferably 5 to 15°C from the point of ease of operation.

The degree of rupturing of the cells can be monitored simply by measuring the optical density (OD). The rupturing should be done to a sufficient extent so that the enzymes are released from the cells.

After finishing rupturing of the cells, the aqueous solution of the enzymes, which are extracted from the cells in the solution, is subjected to heat treatment. The purpose of the heat treatment is to insolubilize or deactivate protein other than tryptophan synthase or tryptophanase. According to this heat treatment, the deactivation of the tryptophanase or tryptophan synthase which is caused by other proteins, such as protease, can be prevented. Some stabilizers may be present during the heat treatment.

As to the stabilizers, it is preferred to use sodium chloride or L-serine. When the heat treatment is conducted in the presence of sodium chloride, an amount above 0.1 wt%, preferably 0.5% to 2.0 wt% is added. In the case of L-serine, an amount of above 1.0 wt%, preferably 2.0 to 10 wt% is added. The addition of these stabilizers is expected to prevent the deactivation of enzymes by heat. The effect of those stabilizers has been described in J.Biol.Chem. 241(66) 980-990.

The heat treatment temperature is above 40°C and below a temperature at which the activity of the tryptophan synthase or tryptophanase is lost. High temperature results in the rapid denaturation of the enzymes. If the temperature is below 40°C, it tends to retard the insolubilization and deactivation of contaminated useless proteins which are of disadvantage to the enzymatic reactions.

The preferred temperature range is 40 to 45°C. The time of heat treatment can be within two hours, and preferably it is sufficient to heat for about 10 minutes. The suspension after finishing the heat treatment is cooled below 30°C, and the residue of the cells (i.e., the debris of the ruptured cells and any nonruptured cells) is removed by appropriate means, such as a centrifugal method to obtain the aqueous enzyme solution. As to the heat treatment, the following procedure can be adopted: the suspension obtained after rupturing the cells is centrifuged to remove the cell residue, and the thus obtained solution is subjected to the heat treatment to precipitate the insoluble substances and the insolubilized substances are removed by a centrifuge. The aqueous enzyme solution is then recovered.

According to the present invention, the recovery rate of the enzyme activity of the aqueous solution is above 90%.

The temperature of the storage of the solution can be below 30°C, and is preferably below 10°C. It is preferable to adjust the pH from 6 to 8. Under these conditions, tryptophan synthase can be stored in the aqueous solution for more than 10 days.

The activity of the aqueous solution is usually 0.1 to 0.2 g tryptophan/enzyme solution ml/Hr. If desired, it is also possible to use the solution for the reaction after being concentrated, such as by ultra-filtration.

The reactions which tryptophan synthase can be used to catalyze are, for example, the reaction between indole and serine to produce L-tryptophan, the reaction between 2-mercaptoethanol and L-serine to produce S- hydroxyethyl cysteine and the reaction between metallic hydrosulfide and L-serine to produce L-cysteine.

As to the reaction between indole and serine, the known method which is conducted in an aqueous solution can be mostly used. For example, the method of feeding the total amount of indole in portions, taking into consideration that indole inhibits the enzyme activity (See, Japanese Kokai Publication No. 58-16692). Indole can be fed from the organic solvent layer to the aqueous layer to utilize the distribution equilibrium (See, GB 2,151,634). It is also possible to use DL-serine or D-serine instead of L-serine in the presence of the serine racemizing enzyme (See, USP 4,335,209). As to the method of feeding indole from the organic solvent layer utilizing the distribution equilibrium mentioned above, such method is described in GB 2,151,634 (the contents of which are incorporated by reference). The L-serine, ammonium sulfate and pyridoxal phosphate, etc., are dissolved or suspended in the water. The L-serine concentration is adjusted 2 to 20 wt%. Indole is dissolved into an organic solvent, such as toluene, and the indole concentration is adjusted to 10 to 40 wt%. Both solutions, the L-serine containing aqueous solution and indole containing solution are charged into the reaction vessel and the molar ratio of the L-serine to the indole is adjusted to 1.0 to 1.1. The amount of aqueous solution of the enzymes extracted from the cells is selected so that the amount of tryptophan obtained is 0.5 to 2.0 g/Hr per 1 g serine. The pH is adjusted to 8.0 to 9.0 by adding alkali, preferably by adding concentrated ammonia solution. The reaction temperature can be 30 to 50°C and the reaction time can be 20 to 60 hours.

According to the present invention, when the reaction is conducted by the above-mentioned manner, the conversion is easily calculated by analyzing the amount of residual indole in the toluene layer. Usually the conversion of the indole to L-tryptophan is 90 to 100%. This conversion rate is comparable to the known process of using whole cells containing the enzyme. In the reaction mass of the aqueous solution thus obtained, above 10 wt% of L-tryptophan is present.

According to the prior known process which uses the intact cells for the source of enzyme in the reaction, it is necessary to remove the used cells by adopting liquid-solid separation methods, so the L-tryptophan in the reaction mass has to be diluted with water to 4 to 5 wt% after the reaction. By the present invention, the cell removal step is not necessary and the large amount of aqueous dilution is not necessary. Therefore, from the point of volume efficiency, it is better to conduct the reaction at the concentration of L-tryptophan above 10 wt%. Prior to applying the purification step, the thus obtained slurry containing L-tryptophan, at ambient temperature (e.g., 20 to 35°C), is contacted with concentrated ammonia solution or ammonia gas to adjust the pH above 10, preferably above 11 to dissolve the L-tryptophan. When the pH is below 10, L-tryptophan in the slurry does not dissolve completely, but above a pH of 10, the solubility of L-tryptophan increases dramatically to above 10 wt% and the slurry becomes an almost homogeneous solution.

As the purification step, according to the prior known process, after removing the insoluble substrates such as the used cells, unreacted indole is removed by means of solvent extraction using toluene for the extractant. If the unreacted indole remains in the final product, the value of the L-tryptophan as feed additive is lost.

In the present invention, the reaction mass forms the homogeneous high L-tryptophan concentration aqueous solution only by adjusting pH above 10 by adding ammonia. The unreacted indole can be removed from the solution by means of solvent extraction, said extraction can be applied to the aqueous solution and separation achieved without using any other steps, such as diluting the solution and removing the insoluble used cells.

Sometimes it is found that a small amount of insoluble substances and colored substances derived from the enzymes are present in the solution. In this case, diatomaceous earth or activated carbon can be added to the solution. Thereafter, the solution is subjected to a solid-liquid separation to remove such impurities. If necessary, further steps, such as contacting the solution with ion-exchange resins or macro-porous resins, can be employed.

Thus, according to the present invention, the L-tryptophan-containing solution can be purified without diluting the solution, as was necessary in previously known processes. Thereafter, the solution is heated to a temperature of 60 to 100°C under atmospheric or reduced pressure to remove the ammonia and to concentrate the solution to a certain extent, for example 10 to 20 wt%. The pH value of the solution becomes 6 to 8, so it is not necessary to neutralize the solution for crystallization of L-tryptophan.

After the concentration step, the crystallization, the filtration and the drying steps follow and the purified L-tryptophan is obtained.

Examples of the invention and comparative examples are described hereinafter.

## EXAMPLES

Example 1: (The preparation of the aqueous enzyme solution)

Escherichia coli MT-10242(FERM BP-20) which is a tryptophan synthase producing strain was inoculated on 100 ml of a culture medium composition described in Table I in a Sakaguchi-flask having a capacity of 500 ml. Culturing was carried out at 35°C for 24 hours. 500 ml of this cultured liquid (six Sakaguchi flasks) was inoculated on 15 liters of a culture medium composition described in Table II in a jar fermenter having a capacity of 30 liters, and cultured at 35.C and pH 6.8 (adjusted by 28% ammonia). Pre-pasteurized 45% glucose solution was fed at intervals in a total amount of about 4.5 kg solution. After culturing 48 hours, 17 liters of the cultured liquid containing the cells which concentration was 30 g dried cells/liter (concentration of the solid substance was 3 wt%) was obtained. Then the cultured liquid was subjected to centrifugal separation to collect cells. The tryptophan synthase activity of the collected cells was 4g-tryptophan/dried cells(g). The collected cells were suspended in a phosphoric acid buffer solution (pH 7.5), with the concentration of the dried cells adjusted to be 30 g/l. 200 ml of said solution was cooled to 5°C and pulverized at a pressure of 11,200 psi (800 Kg/cm²) by a French Pressure Cell Press (SLM AMINCO). The degree of rupturing was measured by optical density (OD) and was 10 as compared to the OD (660nm) of the cell suspension prior to rupturing the cells, which OD is standardized to 100. This OD value of the ruptured cell solution showed that cells were pulverized sufficiently. The suspension after rupturing was subjected to centrifugal separation at not more than 10°C (e.g., by using a high speed centrifuge provided with cooling means) and the residue of the used cells was separated. 1 g of sodium chloride was added to 200 ml of the aqueous solution of the enzymes and the residue of the used cells was separated. The vessel containing the aqueous enzyme solution was dipped in a warm water bath and was maintained at an internal temperature of 40 to 45°C, for 10 minutes. Thereafter, the vessel was cooled rapidly by ice. New insoluble substances were precipitated and again subjected to centrifugal separation by the same type of centrifuge to separate insoluble substances. Finally, 200 ml of the aqueous solution was obtained. The tryptophan synthase activity of the solution was 0.11 g tryptophan/solution ml/Hr. The recovery rate of the activity to the starting cells was 92%. The time dependence of the activity was measured by storing the enzyme solution at 5 to 10°C. After 10 days of storage, the activity was 96 compared to the figure of 100 at the first day which showed good stability. See Table III.

TABLE I

| Bacto-trypton[R] | 10g |
|---|---|
| Bacto-yeast extract[R] | 5g |
| NaCl | 10g |
| distilled water | 1000ml |
| pH was 7.5 | |

## TABLE II

| | |
|---|---|
| $KH_2PO_4$ | 2g |
| $K_2HPO_4$ | 2g |
| $(NH_4)_2SO_4$ | 1.5g |
| $MgSO_4, 7H_2O$ | 2g |
| polypepton | 2g |
| Yeast Extract | 2g |
| $CuCl_2 \, 2H_2O$ | 40mg |
| $MnSO_4 \, 5H_2O$ | 40mg |
| $ZnSO_4 \, 7H_2O$ | 10mg |
| $Na_2MoO_4 \, 2H_2O$ | 10mg |
| $CaCl_2 \, 2H_2O$ | 10mg |
| $CoCl_2 \, 6H_2O$ | 10mg |
| $FeSO_4 \, 7H_2O$ | 10mg |
| $AlCl_3 \, 6H_2O$ | 10mg |
| L-tryptophan (auxotrophic) | 300mg |
| diluted to 1000ml by distilled water (pH 6.8) | |

The activity of tryptophan synthase was tested as follows. The solution containing components described below was used for the reaction. The reaction was carried out for an hour at 35°C. The activity is described by the amount in grams of L-tryptophan produced from L-serine and indole per hour, per ml unit volume of the enzyme solution.

indole     2.0wt%

L-serine     1.8wt%

TritonX-100$^R$     5.0wt%

pyridoxal 5' phosphate     0.01%

ammonia sulfate     2.5%

enzyme solution     0.5ml (diluted 30 times

distilled water     the rest of the amount

pH value     8.5

## Example 2

The experiment was carried out in the same manner as described in Example 1 except that 5 g of L-serine was added in the heat treatment step instead of 1 g of sodium chloride and an aqueous solution of tryptophan synthase was obtained. The tryptophan synthase activity of the solution was 0.11 g tryptophan/enzyme solution ml/Hr, same as Example 1, and the recovery rate of this activity to the starting cells was 92%.

The storage stability of the enzyme solution is shown in the Table III with the result of Example 1.

## Comparative Example 1

The experiment was carried out in the same manner as described in Example 1 except that the heat treatment step of the enzyme solution was omitted. The activity of thus obtained aqueous solution of enzyme was 0.11 g tryptophan synthase/enzyme solution ml/Hr and the value was the same as the Example 1 and 2, but the activity of the solution stored at 5 to 10°C decreases drastically as the time went on. See Table III.

TABLE III

| Storage Date | Enzyme Solution of Example | | Enzyme Solution of Comparative Example |
|---|---|---|---|
| | Example 1 | Example 2 | |
| 0 | 100 | 100 | 100 |
| 1 | 100 | 100 | 95 |
| 2 | 100 | 100 | 90 |
| 3 | 98 | 99 | 80 |
| 5. | 96 | 98 | 70 |

Example 3: (The enzymatic reaction between indole and serine)

160 ml of the aqueous enzyme solution obtained by Example 1, 26.8 g of L-serine and 0.02 g of pyridoxal phosphate were charged in a separate flask having a capacity of 500 ml, and the pH value was adjusted to 8.5 by 1N NaOH, and water was added so that the weight of the aqueous layer was 212.8 g. 26.8 g of indole was dissolved in the 107 g of toluene and this mixture was added to the aqueous layer. The reaction was carried out by agitating for 20 hours in the temperature bath at 35°C. After the reaction had been conducted for 2 to 5 hours, L-tryptophan crystals were formed and accumulated in the reaction vessel and at the end of the reaction the slurry was formed. The content of the remaining unreacted indole was analyzed and the conversion to L-tryptophan from indole was calculated to be 98%. The reaction mass was about 45.8 g and the concentration of L-tryptophan was 13.2 wt%. The reaction vessel had been kept in the temperature bath at 35°C, and then 25% concentration of aqueous ammonia solution was added gradually, the total amount of aqueous ammonia solution was 145 g and the pH value was adjusted 11. L-tryptophan in the water layer was dissolved completely and the toluene layer separated from the water layer and formed the upper layer. The upper layer toluene was recovered by separation and 107 g of fresh toluene was added and the mixture was agitated and was subjected to extraction. Again, toluene was recovered by separation. The indole content in the water layer was below 5 ppm. 350 g of the water layer (L-tryptophan content about 13 wt%) was concentrated to 300 g (L-tryptophan content about 15 wt%). The concentration was carried out by using a rotary evaporator at 60 to 80°C (inner temperature) and at vacuum pressure of about 100 mg/Hg. L-tryptophan precipitated in the concentrated liquid and the pH value was 7.8. The concentrated solution was cooled to 5°C and when crystallization had been conducted for two hours, then crystalline L-tryptophan containing liquid was filtered through Nutsche (Buchner funnel) under reduced pressure. 80.2 g of wet L-tryptophan filter cake was obtained. After drying the cake, 38.4 g of purified L-tryptophan was recovered. The yield of L-tryptophan was 82% to indole. The purity was 99.6%. $[\alpha]_D^{20}$ = -30.9. so it is sufficient enough to meet the official grade of feed additives.

Example 4

The experiment was carried out in the same manner as described in Example 3 except using the aqueous enzyme solution of Example 2 instead of using the solution of Example 1. The yield of L-tryptophan was 80% to indole and the purity was 99.5%

Comparative Example 2

The culturing of the strain was carried out in the same manner as described in Example I and the harvested cells were obtained. The harvested cells (4.4 g dry weight) were charged into a separate flask having a capacity of 500 ml with 26.8 g crystalline L-serine, 0.02 g of pyridoxal phosphate, and the pH value was adjusted to 8.5 by 1N NaOH and water was added so that the weight of the aqueous layer was 212.8 g.

26.8 g of indole was dissolved in 107 g of toluene and this mixture was added to the aqueous layer.

The reaction was carried out by agitating for 20 hours in the temperature bath at 35°C. Soon after starting the reaction, L-tryptophan crystals were formed and precipitated in the reaction vessel.

A slurry was formed, the content of the remaining unreacted indole was analyzed and the conversion to L-tryptophan from indole was calculated at 95%. Then 920 g of water was added to the reaction mass and the concentration of L-tryptophan was adjusted to 4 wt%. In this state, the aqueous layer and the toluene layer did not separate because of a floating slurry of the cells and it was impossible to separate the two layers. The solution was heated with agitation and toluene was recovered with water by utilizing azeotropic distillation. The remaining water layer was about 1,010 g in weight and contained 43.5 g of L-tryptophan and cooled to 40°C. Then 5 g of the concentrated sulfuric acid was added and the pH value was adjusted to 4.0. 10 g of purified activated carbon was added and heated at 90 to 95°C for an hour with agitation to aggregate the cells around the activated carbon which operated as a core material. The treated solution was filtered through Nutsche (Buchner funnel) by heating and about 1000 g of the filtrate was obtained. 36.9 g of L-tryptophan was contained in the filtrate. 107 g of toluene was added to the filtrate and the mixture was heated at 50 to 80°C and agitated to wash the filtrate. The toluene was then recovered by separation. The indole content in the water layer was below 5 ppm. 1000 g of the water layer was concentrated to 250 g (L-tryptophan content was about 15 wt%). The concentration was carried out by using rotary evaporate at 60 to 80°C (inner temperature) and at a vacuum pressure of about 100 mm Hg. The pH value was adjusted to 5.6 and L-tryptophan was precipitated. The concentrated solution was treated in the same manner described in Example 3, so it was cooled to 5°C and after crystallization had been conducted for two hours, the crystalline L-tryptophan containing liquid was filtered through Nutsche (Buchner funnel) under reduced pressure.

57.0 g of wet L-tryptophan filter cake was obtained. After drying the cake, 29.5 g of L-tryptophan was recovered. The yield of L-tryptophan was 63.0% to indole, the purity was 99.4% and $[\alpha]_D^{20} = -30.3$.

Example 5

Instead of Escherichia Coli MT-10242 (FERM BP-20) used in Example 3, Aerobacter aerogenes IFO 3317, a tryptophanase-producing strain was inoculated on 200 ml of a culture medium composition described in Table I in a flask having a capacity of 500 ml. Culturing was carried out at 30°C for 24 hours. 600 ml of thus cultured liquid was inoculated on 15 liters of a culture medium composition described in the Table IV in a jar fermenter having a capacity of 30 liters, and cultured at 30°C and a pH value of 6.8 (adjusted by 28% aqueous ammonia solution). Pre-pasteurized 45% glucose solution was added at intervals. After culturing 46 hours, 17 liters of the cultured liquid containing the cells which concentration was 20 g/l (as dried cells) was obtained. The cultured liquid was subjected to centrifugal separation to harvest cells. The harvested cells were subjected to rupturing by a French Pressure Cell Press. The cell residue were separated, and subjected to the heat treatment in the same manner described in Example 1.

The reaction of indole and serine was carried out in the same manner as described in Example 3 except using the 150 ml of the aqueous solution of enzymes obtained by the above-mentioned process. The conversion of indole to tryptophan was 94%. After concentration and crystallization, 72 g of tryptophan filter cake was obtained. Finally, after drying the cake, 35 g of purified L-tryptophan was obtained. The yield was 75% to indole and the purity was 99.2%.

TABLE IV

| | |
|---|---|
| $KH_2PO_4$ | 2g |
| $K_2HPO_4$ | 2g |
| $(NH_4)_2SO_4$ | 2g |
| $MgSO_4 7H_2O$ | 2g |
| polypepton | 4g |
| yeast extract | 4g |
| $CuCL_2 2H_2O$ | 40mg |
| $MnSO_4 5H_2O$ | 40mg |
| $ZnSO_4 7H_2O$ | 10mg |
| $Na_2MoO_4 2H_2O$ | 10mg |
| $H_3BO_3$ | 10mg |
| $Cr_2Cl_2 2H_2O$ | 10mg |
| $CoCl_2$ | 10mg |
| $FeSO_4$ | 10mg |
| $AlCl_3$ | 10mg |
| L-tryptophan | 20mg |
| distilled water | 1000ml |

## Claims

1. A method of preparing L-tryptophan comprising:

a) reacting indole and serine to form L-tryptophan in a reaction mixture containing an aqueous enzyme solution that has been extracted from cells;

b) subsequent to the reaction and the formation of the L-tryptophan, adding ammonia to the reaction mixture to adjust the pH to at least about 10 so as to dissolve the L-tryptophan and form a solution containing the L-tryptophan;

c) separating the solution containing the L-tryptophan; and

d) heating the solution to remove ammonia therefrom and to concentrate the L-tryptophan in the solution.

2. The method of claim 1 wherein the concentration in the solution prior to heating is at least about 10% by weight.

3. The method of claim 1 wherein the indole is added to the reaction mixture in the form of a solution in an organic solvent and the residual organic solvent forms a separate layer from said solution upon the addition of ammonia.

4. The method of claim 3 wherein the solution is separated from the reaction mixture and is subjected to solvent extraction to remove unreacted indole.

5. The method of claim 4 wherein the organic solvent is toluene.

6. The method of claim 1 wherein the aqueous enzyme solution contains tryptophan synthase.

7. The method of claim 1 wherein the aqueous enzyme solution contains tryptophanase.

8. The method of claim 1 wherein the ammonia is in the form of aqua ammonia or ammonia gas.

9. The method of claim 1 wherein ammonia is added to adjust the pH to at least about 11.

10. The method of claim 1 wherein the solution is heated to from about 60° to about 100°C.

11. The method of claim 1 wherein the separated solution is subjected to a liquid-solid separation step to remove small particles from the solution.

12. A method of preparing an aqueous enzyme solution of tryptophan synthase or tryptophanase comprising:

a) culturing microorganisms capable of producing tryptophan synthase or tryptophanase;

b) rupturing the microorganisms to extract tryptophan synthase or tryptophanase therefrom;

c) heating the extracted tryptophan synthase or tryptophanase to a temperature above about 40°C for a time sufficient to deactivate or insolubilize proteins other than the tryptophan synthase or tryptophanase; and

9

d) separating the extracted tryptophan synthase or tryptophanase in the form of an aqueous solution from the insolubilized protein.

13. The method of claim 12 wherein the rupturing is conducted at a temperature below 30°C.

14. The method of claim 12 wherein the heat treatment is conducted at a temperature of from about 40° to about 50°C.

15. The method of claim 12 wherein the heat treatment is conducted in the presence of a stabilizer.

16. The method of claim 15 wherein the stabilizer is sodium chloride in an amount above about 0.1% by weight.

17. The method of claim 15 wherein the stabilizer is L-serine in an amount above about 1.0% by weight.

18. The method of claim 12 wherein the ruptured microorganisms are separated from the tryptophan synthase or tryptophanase prior to heating.

19. The method of claim 18 wherein the ruptured microorganisms are separated by centrifugation.

20. The method of claim 12 wherein the tryptophan synthase or tryptophanase is separated from the insolubilized protein by centrifugation.